# EUROPEAN PATENT APPLICATION

(11) **EP 3 904 531 A1**
(43) Date of publication of application: **03.11.2021**
(21) Application number: 20171920.0
(22) Date of filing: 28.04.2020
(51) Int. Cl.: C12Q 1/6806

(54) **METHOD FOR SINGLE CELL ISOLATION AND RNA EXTRACTION FROM WOODY TISSUES**

(30) Priority: 27.04.2020 PT 2020116298
(71) Applicant: Cebal - Centro De Biotecnologia Agrícola E Agro-Alimentar Do Alentejo, 7801-908 Beja (PT)
(72) Inventor: Costa Santos Pires, Rita Alexandra, 6300-551 Guarda (PT); Costa Capote, Tiago, 7800-554 Beja (PT); Sarilho Ferro, Ana Margarida, 7800-554 Beja (PT); Bota Marum, Liliana Maria, 2660-445 Santo António dos Cavaleiros (PT)
(74) Representative: Ferreira, Maria Silvina

(57) **Abstract**

The present patent application discloses a method for single cell isolation and RNA extraction in woody plants' tissues, while preserving the viability of the nucleic acids for further analysis. The method comprises, collecting a woody plant tissue sample, snap-freezing and embedding the sample in Optimal Cutting Temperature (OCT) freezing medium, sectioning of the frozen samples into sections of 10 to 20 µm of thickness, fixation and dehydration of plant sections, isolation of single cells by laser microdissection, RNA extraction from isolated single-cells, wherein the RNA extraction comprises the use of a lysis buffer comprising stabilizers.

Thus, it is disclosed here for the first time a method for single cell isolation and RNA extraction of isolated woody tissues comprising xylem and suberized cells while still maintaining RNA integrity for RNA sequence analysis.

## Description

### Technical field

This application relates to a method for single cell isolation and RNA extraction from woody tissues, for RNA-Seq analysis.

### Background art

A multicellular organism, such as plant species, is a group of structures composed by different types of cells that exhibit specific genetic, protein and metabolites profiles. The information acquired from whole organ analysis can mask valuable data at cellular level. Advances in isolation of individual cell populations have made possible cell or tissue type-specific transcriptome profiles, with specific biological functions, revealing complex gene networks. Some methodologies for isolating cells- or tissues-types have been developed to make such analysis more specific and precise as possible, enhancing the detection of low-abundance transcripts that are expressed in only few and specialized cells. The Laser Microdissection (LM) is a very precise technology that allows to isolate cells or tissue of interest from a heterogeneous cell population, using a microscope to visualize a tissue section and a laser beam to separate the target cells of surrounding tissue. The LM technique was originally developed for animal tissues (Emmert-Buck et al., 1996; Isenberg et al., 1976) in which the absent of cell wall favors the RNA isolation. Later, the use of this technology in plants cells was first reported by Asano et al. (2002) in rice. Since then, research have been made with different purposes and using several tissues and plant models such as Arabidopsis (Anjam et al., 2016; Sakai et al., 2018), rice (Shiono et al., 2014; Takahashi et al., 2010), maize (Ohtsu et al., 2007) and tomato (Martin et al., 2016). However, there are only few studies of LM application in woody species (Abbott et al., 2010; Cañas et al., 2014; Larisch et al., 2012; Luchi et al., 2012) and consequently in xylemic (Blokhina et al., 2017) and suberized cells, in which the level of suberization is in lead-off formation (Teixeira et al., 2018). The wood and non-wood forest materials, like cork, are important forest materials with a high economic and social world impact. The identification of biomarkers in species with a long life cycle, like forest species, confers an additional advantage in terms of early selection of traits, such as wood or cork quality, that manifest only in adult stage. Single-cell RNA sequencing (RNA-seq) is a powerful tool to identify these potential biomarkers, highlighting the importance of RNA samples homogeneity.

The isolation of single cells from plant tissues had additional challenges such as rigid cell walls, large central vacuoles and large intercellular spaces, which can comprise the integrity of the tissues of interest. The structural tissue of the woody plants, the wood, is consisted mainly by xylem cells, with cell walls of cellulose and lignin, difficulting the application of laser microdissection. Additionally, suberized cells present in periderms are characterized by the cell wall deposition of suberin, conferring an environment barrier against water lost and protection against pathogens. The outer bark from *Quercus suber,* known as cork, is an example of this protective tissue made of suberized cells. The suberin confers unique proprieties to the cork, like impermeability and sound insulation, highlighting the economic and commercial interest of this tissue (Graça, 2015). Therefore, the suberin deposition can also conferring additional challenges for the preparation of Single-Cell RNA Samples, for Next Generation Sequencing.

The extraction of high purity and integrity RNA is a requirement for molecular analysis such as gene expression, microarrays, cDNA synthesis and RNA sequencing. However, RNA is more susceptible to degradation compared to DNA. According to Morrogh et al. (2007), the RNA quality can be compromised up to 75% by inappropriate/insufficient tissue preparation, during single cell isolation by LM. Several adaptions on the protocol can be made, in order to maximize the yield and quality of total RNA isolated from such difficult tissue of woody plants. Until now, no simple protocol was made available to isolate undegraded total RNA from both xylem and suberized tissues for Next Generation Sequencing.

### Summary

The present patent application relates to a method for single cell isolation and RNA extraction from woody tissues, for RNA-Seq analysis.

Therefore, it is disclosed herein a method for single cell isolation and RNA extraction in woody plants' tissues, wherein the method comprises the following steps:
- Collection of a woody plant tissue sample;
- Snap-freezing the sample in liquid nitrogen and embedding the sample in Optimal Cutting Temperature (OCT) freezing medium;
- Sectioning of the frozen samples into sections of 10 to 20 µm of thickness, at -20 to -27 °C;
- Fixation and dehydration of plant sections;
- Isolation of single cells by laser microdissection during a period of up to 180 min;
- RNA Extraction from isolated single-cells, wherein the RNA extraction comprises the use of a lysis buffer comprising stabilizers.

In one preferable embodiment, the OCT freezing medium comprises 10 to 12% Polyvinyl alcohol and 3,5 to 5% Polyethylene glycol, preferably 10,24% Polyvinyl alcohol and 4,26% Polyethylene glycol.

In one embodiment, the laser microdissection is performed on PET-membrane slides.

In one embodiment, the frozen samples are sectioned at -25 °C.

In one embodiment the frozen samples are sectioned into sections of 14 µm of thickness.

In one embodiment, the period of laser microdissection session for collecting cells should not be more than 40 minutes.

In one embodiment, isolation of single cells by laser microdissection is performed until 18000 to 125000 cells are isolated.

In one embodiment, the stabilizer in the lysis buffer used in the RNA extraction step comprises 1,5 to 5% of polyvinylpyrrolidone (PVP).

### Disclosure/Detailed Description

The present patent application discloses an optimized method for single cell isolation and RNA extraction in woody plants, while preserving the viability of the nucleic acids for further analysis. The preparation of the biological sample include embedding, sectioning, fixing, dehydrating, microdissecting, isolating nucleic acids and/or inactivating proteins (including nucleases) . At the end of each extraction process, a capillary electrophoresis is performed to check the quality and concentration of total RNA. The cDNA libraries are constructed from the total RNA samples, and thereafter the samples are sequenced by Illumina platform.

The method of the present patent application comprises the following steps:
A. Collection of a woody plant tissue sample;
B. Snap-freezing the sample in liquid nitrogen and embedding the sample in a OCT freezing medium;
C. Sectioning of the frozen samples in Cryostat Microtome, using 10 to 20 µm of thickness, at -20 to -27 °C;
D. Fixation and dehydration of plant sections;
E. Isolation of single cells by laser microdissection, using 18000 to 125.000 cells, during a period session no more than 180 min;
F. RNA Extraction from isolated single-cells, wherein the RNA extraction comprises the use of a lysis buffer comprising stabilizers.
A) The tissue collection is proceed quickly and using all procedures in RNase-free conditions to minimize RNA degradation.
B) The tissue is "snap-frozen" in liquid nitrogen inactivating the RNAses and then embedded with an optimal cutting temperature embedding(OCT)medium for frozen tissue specimens, wherein the OCT medium comprises a water-soluble blend of 10,24% Polyvinyl alcohol, 4,26% Polyethylene glycol and 85,5% nonreactive ingredients that provides a convenient specimen matrix for cryostat sectioning at low temperatures, like -25°C.
C) In a cryostat, tissue sections can be cut into 3-20 micrometer in thickness (a process herein called "sectioning" the tissue) and mounted onto glass or PET-membrane microscope slides for microscopic purposes, like histological examination and/or laser microdissection. The slides are sterilized previously by UV radiation for 30min.
D) Fixation step is performed to stabilize the cell contents and to preserve histological integrity. It helps also to remove the OCT medium and the sections adhesions to the microscopic slides. The dehydration step avoids problems during laser cutting due to the formation of small water bubbles that blocks the correct tissue adhesion to the slide.
E) The next step is isolate the cells or tissue of interest using Laser Microdissection (LM) previously cleaned with RNase AWAY. Briefly, LM technique uses a microscope to visualize a tissue section and a laser beam to separate the target cells from the surrounding tissue. The detection of desired cells is made directly based on its morphology and/or histological staining.
F) After microdissection, biomolecules (DNA, RNA, proteins and metabolites) can be extracted from the harvested cells for a variety of downstream applications, such as RT-PCR, gene expression studies, microarrays, next generation sequencing, proteomic and metabolic analysis. Preferentially, total cellular RNA extraction can be performed using a pico-scale purification column membrane from single cell Kits.

The low RNA yield obtained from woody tissues can be due to RNA degradations or to the presence of contaminants, like phenolics and polysaccharides. To avoid it, the present invention comprises the optimization of C, E and F steps concerning the histological section thickness, slides membrane type, the single cell collection time, the number of isolated cells during the LM session and the lysis buffer composition used during the RNA extraction.

In step C, the best histological section thickness obtained during cryostats sectioning was 14 µm. The thickness obtained was a commitment between a good cell structure integrity and the higher number of cells needed, and the higher laser power used to cut the woody tissues.
Moreover, the PET-membrane slides were the best option to use in the woody tissues, in alternative to glass slides. The glass slides were not effective during LM sessions in cutting the woody tissues, because the laser power resulted in diffusion of the beam and decrease laser cutting efficacy. The PET-membrane slides eliminated the need for glass support, facilitating the use of increased laser power.

In the present invention, the single cell collection time during the LM session (step E) was tested to avoid RNA degradation. This assay is described in Example 1. The period of LM session for collecting cells should not be more than 40 minutes.

Additionally, the RNA yield depends also on the plant cell type isolated during LM session. Taking into account the specificities of the xylem and suberized tissues, the number of cells isolated by LM was also analysed, as described in Example 2. The number of isolated cells should never be less than 18000.

The standard RNA extraction procedure describe in step F usually uses a lysis buffer that allows cell membrane disruption and inactivation of intracellular nucleases, like RNAses, stabilizing the cell contents. However, the woody plants have a high percentage of secondary metabolites, such as total polyphenols, condensed tannins, and free and esterified phenolic acids. Polyphenolic compounds, which has been described as having affinity to nucleic acids, forming complexes, are released at different levels in the woody tissues, like in *Quercus* spp.
To overcome this problem, in the present invention, stabilizing solutions were used to eliminate possible contaminants in the RNA samples.
The use of RNA stabilizers, like polyvinylpyrrolidone (PVP), bind to the polyphenolic compounds, avoiding the inhibitory effects of this compounds, and consequently improving the yield of the RNA extraction.
In the Example 3, it is shown that the addition of RNA stabilizers to the lysis buffer improves the RNA yield and integrity.

### Examples

The following experiments (Examples 1, 2, 3 and 4) were performed to achieve a simple and efficient method for RNA extraction from single cells of woody tissues. Branches of cork oak (*Quercus suber*) were snap-frozen in liquid nitrogen after harvesting, embedded in OCT, followed by sectioning into 14-micrometer sections at -25°C in a cryostat (CryoStar NX50 Cryostat, a product of Thermo Scientific Inc.). The sections were mounted on PET-membrane slides (sterilized earlier by UV radiation for 20min), and fixated in ice-cold 70% ethanol during 2 min, with some drops covering the all sections. Then the 70% ethanol was replaced by ice-cold 100% ethanol, facilitating the adhesion of sections. The slides were completely air-dried at room temperature (RT) before the microdissection. Any humidity in section can subsequently compromise the laser cuttings.

To eliminate the risk of RNA degradation, all surfaces and instruments were properly decontaminated with RNase cleaner solution (product of Nzytech Inc.). When it was possible the instruments were incubated under UV radiation for 30 min or autoclaved.

The area of interest selected was dissected, using a PALM Zeiss Microbeam Microscope, at 20x magnification and with the following settings: laser energy range 69 to 75, a LPC (laser pressure catapulting) energy value from 94 to 100, delta 25, a laser focus and a LPC focus delta of 59 and a speed cut of 10. The microdissections were catapulted into an adhesive cap tube (AdhesiveCap 500 clear, product of Carl Zeiss Inc.).

After the collection, 10 µL of lysis buffer (PicoPure™ RNA Isolation Kit, product of Thermo Fisher Scientific Inc.) were added to the cap. The tubes were vortexed and incubated for 30 minutes at 42°C, as the manufacturer's instructions; then centrifuged at 800g for 2min. The samples were stored at -80°C (5 days maximum), until further RNA extraction steps.

In all experiments, total RNA extraction was carried out with the RNA isolation kit prepared to single cell RNA isolation (PicoPure™ RNA Isolation Kit, product of Thermo Fisher Scientific Inc.), according to the manufacturer's instructions. To eliminate genomic DNA, the RNA extracted was treated with DNase (RNase-Free DNase Set, product of QIAGEN Inc.) following the manufacturer's instructions. At the end of each extraction process, a parallel capillary electrophoresis using LabChip technology (Fragment Analyser System) was performed to check the quality and quantity of RNA.

For each assay, at least three to six replicates were used. Data were analyzed by one-way analysis of variance (ANOVA) and t-student test. Post-hoc analyses were performed using Tukey's HSD methods. Significance level was set at 0.05. All statistical analyses were performed using STATISTICA (data analysis software system), version 7. Data are presented as mean ± standard deviation.

### Example 1 - Influence of single cell collection time

In the present example, the influence of single cell collection time, in a laser microdissection session, on the quantity and quality of extracted RNA was evaluated. Three different times were analyzed: 0 minutes (the lysis buffer was added right after the sectioning), 40 minutes and 120 minutes. For each sample, total RNA from 18 000 cells (three whole sections) was extracted as mentioned above.

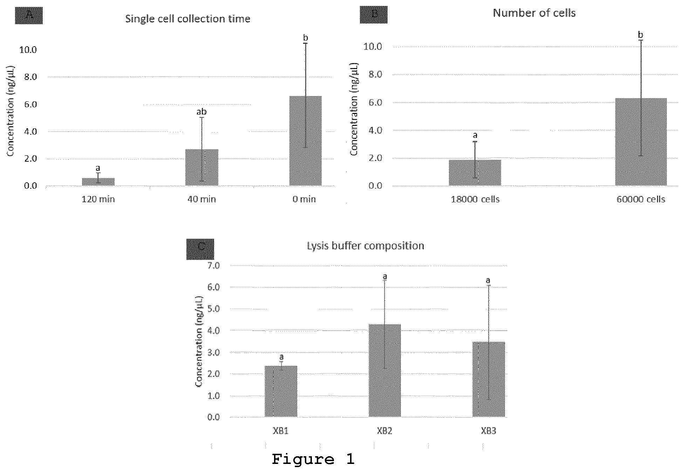

Following the method of described herein, the yield of RNA increases significantly using when the collection time is shorter (0 and 40 min) (Fig.1A). The data validated that the most significant difference is between 0 min (i.e. addition of lysis buffer immediately after sectioning) and 120min (time for performing laser microdissection session) (p<0.05). However, no statistical differences were detected between the times 0 min and 40 min. Regardless of that, there is an effect of the collection time, on the concentration of RNA. The period for harvesting the cells of interest and the addition of lysis buffer should not be more than 40 minutes. The RNA integrity, expressed by RQN parameter (RNA Quality Number), was also improved using a lower collection time.

### Example 2 - Influence of the number of isolated cells

In this example, the influence of the number of isolated cells from woody tissues on the RNA extraction yield was analyzed. Extraction of RNA from approximately 18000 cells and 60000 cells was conducted as described above.

Data presented in Figure 1B showed a significant difference on RNA concentration between the samples isolated from 18000 and 60000 cells (p<0.05). The number of harvested cells should be a compromise between the enough quantity of RNA for the chosen downstream application and the smallest time of tissue collection possible.

### Example 3 - Influence of lysis buffer composition

In the following example, the effect of lysis buffer composition on RNA quality and concentration was studied. The single cell isolation of 18000 cells and RNA extraction procedures were conducted as described above. Two different stabilizers solutions were added to the lysis buffer (XB) with the purpose of improving the yield and quality of isolated RNA. The Plant RNA Isolation Aid (product of Thermo Fisher Scientific Inc.) (XB2) and a solution 2% of polyvinylpyrrolidone (PVP) (XB3) were added to the lysis buffer. The lysis buffer XB present in the RNA extraction kit was used as control (XB1).

A higher concentration of undegraded RNA was obtained when the RNA stabilizers were added to the lysis buffer. Besides no significant difference of RNA amount was observed between conditions (Fig.1C), the RNA stabilizers increased the RNA yield from 2ng/µL to 4.3 ng/µL or 3.4 ng/µL, when XB2 (Plant RNA Isolation Aid) or XB3 (PVP) were added, respectively. Moreover, the stabilizers contributed to a significant improvement in the RNA integrity, which is expressed by the RNA Quality Number. The RQN were duplicate when the XB2 was used.

### Example 4 - RNA extraction for RNA sequencing

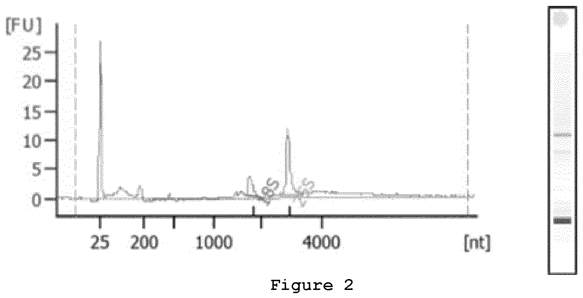

In the following example, single cells from two different tissue of cork oak (xylem and phellogen) were isolated using laser microdissection and total RNA was extracted following the method described in the present invention. The sections were cut as mentioned above and mounted onto PET-membrane slide. A 40 minutes laser microdissection session were performed in order to collect the single cells. Then, total RNA was extracted (Fig.2) from the harvested tissue using the protocol present in the invention, using stabilizer solution XB2. At the final stage, RNA was converted to cDNA through SMART-seq procedure (Picelli et al., 2014), small fragments of cDNA were created by Nextera fragmentation (Baym et al., 2015) and sequencing through Illumina HiSeq 2500 process.

Several features are described hereafter that can each be used independently of one another or with any combination of the other features. However, any individual feature might not address any of the problems discussed above or might only address one of the problems discussed above. Some of the problems discussed above might not be fully addressed by any of the features described herein. Although headings are provided, information related to a particular heading, but not found in the section having that heading, may also be found elsewhere in the specification.

### Brief description of drawings

For easier understanding of this application, figures are attached in the annex that represent the preferred forms of implementation which nevertheless are not intended to limit the technique disclosed herein.
Figure 1 shows the mean RNA concentration (ng/µL). Values are mean ± SD (SD-standard deviation of the mean). **A.** Data obtained for the single cell collection times assay, described in Example 1. Means (n =4) followed by different letters are statistically significant different for α=0.05 as evaluated by ANOVA (F=6.33; p< 0.05), followed by Tukey's HSD multiple comparisons test; **B.** Data obtained for the number of cells assay, described in Example 2. Means (n =5-6) followed by different letters are statistically significant different for α=0.05 as evaluated by t-student test (t-value=2.47; p < 0.05); **C.** Data obtained for lysis buffer composition studied, described in Example 3. Means (n =3-4) followed by same letter aren't statistically significant different for α=0.05 as evaluated by ANOVA (F=0.73; p > 0.05).
Figure 2 illustrates an example of electropherogram and a digital gel of total RNA extracted from cork oak tissue using the protocol describe in this invention. RNA was analyzed by capillary electrophoresis using LabChip technology (Fragment Analyser System, Agilent). The observed peaks corresponding to 18S and 28S rRNA. The sample total concentration was estimated at 137 pg/µl and the RQN number at 8.6.

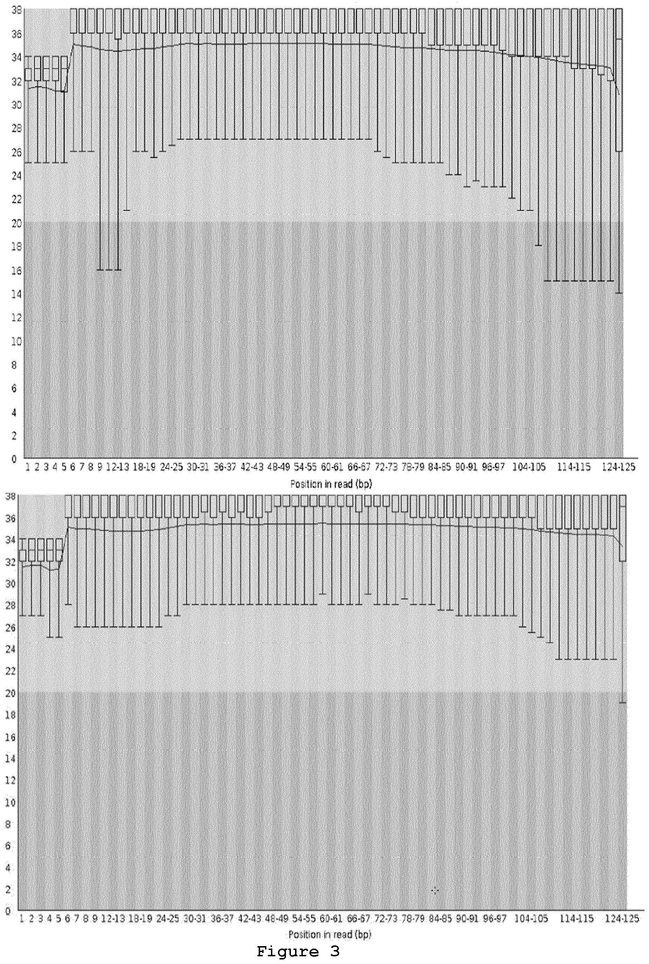

Figure 3 shows a graph concerning RNA sequencing quality scores across all bases (Sanger/Illumina 1.9 encoding) for a sample of xylemic (A) and phellogen/suberized (B) cells collected using laser microdissection, obtained in Example 4. On Y-axis is the quality scores (Q score) and X-axis the position in read (bp). Q score indicates the probability that a given base is called incorrectly by the sequencer. The higher the score the better the base call. In the samples analyzed, most of the reads have a Q score >30 which suggest an accurate sequencing for both xylem and suberized samples.

### Description of the embodiments

Now, preferred embodiments of the present application will be described in detail. However, these are not intended to limit the scope of this application.

The present patent application discloses a method for single cell isolation and RNA extraction in woody plants' tissues, wherein the method comprises the following steps:
- Collection of a woody plant tissue sample;
- Snap-freezing the sample in liquid nitrogen and embedding the sample in Optimal Cutting Temperature (OCT) freezing medium;
- Sectioning of the frozen samples into sections of 10 to 20 µm of thickness, at -20 to -27 °C;
- Fixation and dehydration of plant sections;
- Isolation of single cells by laser microdissection during a period of up to 180 min;
- RNA Extraction from isolated single-cells, wherein the RNA extraction comprises the use of a lysis buffer comprising stabilizers.

In one preferable embodiment, the OCT freezing medium comprises 10 to 12% Polyvinyl alcohol and 3,5 to 5% Polyethylene glycol, preferably 10,24% Polyvinyl alcohol and 4,26% Polyethylene glycol.

In one embodiment, the laser microdissection is performed on PET-membrane slides.

In one embodiment, the frozen samples are sectioned at -25 °C.

In one embodiment the frozen samples are sectioned into sections of 14 µm of thickness.

In one embodiment, the period of laser microdissection session for collecting cells should not be more than 40 minutes.

In one embodiment, isolation of single cells by laser microdissection is performed until 18000 to 125000 cells are isolated.

In one embodiment, the stabilizer in the lysis buffer used in the RNA extraction step comprises 1,5 to 5% of polyvinylpyrrolidone (PVP).

This description is of course not in any way restricted to the forms of implementation presented herein and any person with an average knowledge of the area can provide many possibilities for modification thereof without departing from the general idea as defined by the claims. The preferred forms of implementation described above can obviously be combined with each other. The following claims further define the preferred forms of implementation.

### References

Abbott, E., Hall, D., Hamberger, B., & Bohlmann, J. (2010). Laser microdissection of conifer stem tissues: Isolation and analysis of high quality RNA, terpene synthase enzyme activity and terpenoid metabolites from resin ducts and cambial zone tissue of white spruce (Picea glauca) . BMC Plant Biology, 10, 1-16. https://doi.org/10.1186/1471-2229-10-106 Alonso, M., Rozados, M. J., Vega, J. A., Perez-Gorostiaga, P., Cuiñas, P., Fontúrbel, M. T., & Fernandez, C. (2002). Biochemical responses of Pinus pinaster trees to fire-induced trunk girdling and crown scorch: Secondary metabolites and pigments as needle chemical indicators. Journal of Chemical Ecology, 28(4), 687-700. https://doi.org/10.1023/A:1015276423880
Anjam, M. S., Ludwig, Y., Hochholdinger, F., Miyaura, C., Inada, M., Siddique, S., & Grundler, F. M. W. (2016). An improved procedure for isolation of high-quality RNA from nematode-infected Arabidopsis roots through laser capture microdissection. Plant Methods, 12(1), 1-9. https://doi.org/10.1186/s13007-016-0123-9
Asano, T., Masumura, T., Kusano, H., Kikuchi, S., Kurita, A., Shimada, H., & Kadowaki, K. I. (2002). Construction of a specialized cDNA library from plant cells isolated by laser capture microdissection: Toward comprehensive analysis of the genes expressed in the rice phloem. Plant Journal, 32(3), 401-408. https://doi.org/10.1046/j.1365-313X.2002.01423.x Baym, M., Kryazhimskiy, S., Lieberman, T. D., Chung, H., Desai, M. M., & Kishony, R. K. (2015). Inexpensive multiplexed library preparation for megabase-sized genomes. PLoS ONE, 10(5), 1-15. https://doi.org/10.1371/journal.pone.0128036
Blokhina, O., Valerio, C., Sokolowska, K., Zhao, L., Kärkönen, A., Niittylä, T., & Fagerstedt, K. (2017). Laser capture microdissection protocol for xylem tissues of woody plants. Frontiers in Plant Science, 7(January), 1-14. https://doi.org/10.3389/fpls.2016.01965
Cañas, R. A., Canales, J., Gómez-Maldonado, J., Ávila, C., & Cánovas, F. M. (2014). Transcriptome analysis in maritime pine using laser capture microdissection and 454 pyrosequencing. Tree Physiology, 34(11), 1278-1288. https://doi.org/10.1093/treephys/tpt113
Castillo-Mendoza, E., Salinas-Sanchez, D., Valencia-Cuevas, L., Zamilpa, A., & Tovar-Sánchez, E. (2018). Natural hybridisation among Quercus glabrescens, Q. rugosa and Q. obtusata (Fagaceae): Microsatellites and secondary metabolites markers. Plant Biology, 0-1. https://doi.org/10.1111/plb.12899
Emmert-Buck, M. R., Bonner, R. F., Smith, P. D., Chuaqui, R. F., Zhuang, Z., Goldstein, S. R., Weiss, R. A., & Liotta, L. A. (1996). Laser Capture Microdissection. Cell Biology, Four-Volume Set, 274(November), 998-1001. https://doi.org/10.1016/B978-012164730-8/50162-3
Goué, N., Noel-Boizot, N., Vallance, M., Magel, E., & Label, P. (2012). Microdissection to isolate vascular cambium cells in poplar. Silva Fennica, 46(1), 5-16. https://doi.org/10.14214/sf.62
Graça, J. (2015). Suberin: The biopolyester at the frontier of plants. Frontiers in Chemistry, 3(OCT), 1-11. https://doi.org/10.3389/fchem.2015.00062
Isenberg, G., Bielser, W., Meier-Ruge, W., & Remy, E. (1976). Cell surgery by laser micro-dissection: a preparative method. Journal of Microscopy, 107, 19-24. https://doi.org/10.1126/science.286.5444.1498
Koonjul, P. K., Brandt, W. F., Farrant, J. M., & Lindsey, G. G. (1999). Inclusion of polyvinylpyrrolidone in the polymerase chain reaction reverses the inhibitory effects of polyphenolic contamination of RNA. Nucleic Acids Research, 27(3), 915-916. https://doi.org/10.1093/nar/27.3.915 Larisch, C., Dittrich, M., Wildhagen, H., Lautner, S., Fromm, J., Polle, A., Hedrich, R., Rennenberg, H., Müller, T., & Ache, P. (2012). Poplar wood rays are involved in seasonal remodeling of tree physiology. Plant Physiology, 160(3), 1515-1529. https://doi.org/10.1104/pp.112.202291
Luchi, N., Capretti, P., Fossdal, C. G., Pazzagli, M., & Pinzani, P. (2012). Laser microdissection on Norway spruce bark tissue: A suitable protocol for subsequent real-time reverse transcription-polymerase chain reaction (RT-PCR) analysis. Plant Biosystems, 146(1), 92-98. https://doi.org/10.1080/11263504.2010.505017
Martin, L. B. B., Nicolas, P., Matas, A. J., Shinozaki, Y., Catalá, C., & Rose, J. K. C. (2016). Laser microdissection of tomato fruit cell and tissue types for transcriptome profiling. Nature Protocols, 11(12), 2376-2388. https://doi.org/10.1038/nprot.2016.146
Morrogh, M., Olvera, N., Bogomolniy, F., Borgen, P. I., & King, T. A. (2007). Tissue preparation for laser capture microdissection and RNA extraction from fresh frozen breast tissue. BioTechniques, 43(1), 41-48. https://doi.org/10.2144/000112497
Ohtsu, K., Smith, M. B., Emrich, S. J., Borsuk, L. A., Zhou, R., Chen, T., Zhang, X., Timmermans, M. C. P., Beck, J., Buckner, B., Janick-Buckner, D., Nettleton, D., Scanlon, M. J., & Schnable, P. S. (2007). Global gene expression analysis of the shoot apical meristem of maize (Zea mays L.). Plant Journal, 52(3), 391-404. https://doi.org/10.1111/j.1365-313X.2007.03244.x
Picelli, S., Faridani, O. R., Björklund, Å. K., Winberg, G., Sagasser, S., & Sandberg, R. (2014). Full-length RNA-seq from single cells using Smart-seq2. Nature Protocols, 9(1), 171-181. https://doi.org/10.1038/nprot.2014.006
Sakai, K., Taconnat, L., Borrega, N., Yansouni, J., Brunaud, V., Paysant-Le Roux, C., Delannoy, E., Martin Magniette, M. L., Lepiniec, L., Faure, J. D., Balzergue, S., & Dubreucq, B. (2018). Combining laser-assisted microdissection (LAM) and RNA-seq allows to perform a comprehensive transcriptomic analysis of epidermal cells of Arabidopsis embryo. Plant Methods, 14(1), 1-12. https://doi.org/10.1186/s13007-018-0275-x
Shiono, K., Yamauchi, T., Yamazaki, S., Mohanty, B., Imran Malik, A., Nagamura, Y., Nishizawa, N. K., Tsutsumi, N., Colmer, T. D., & Nakazono, M. (2014). Microarray analysis of laser-microdissected tissues indicates the biosynthesis of suberin in the outer part of roots during formation of a barrier to radial oxygen loss in rice (Oryza sativa). Journal of Experimental Botany, 65(17), 4795-4806. https://doi.org/10.1093/jxb/eru235
Takahashi, H., Kamakura, H., Sato, Y., Shiono, K., Abiko, T., Tsutsumi, N., Nagamura, Y., Nishizawa, N. K., & Nakazono, M. (2010). A method for obtaining high quality RNA from paraffin sections of plant tissues by laser microdissection. Journal of Plant Research, 123(6), 807-813. https://doi.org/10.1007/s10265-010-0319-4
Teixeira, R. T., Fortes, A. M., Bai, H., Pinheiro, C., & Pereira, H. (2018). Transcriptional profiling of cork oak phellogenic cells isolated by laser microdissection. Planta, 247(2), 317-338. https://doi.org/10.1007/s00425-017-2786-5

## Claims

1. Method for single cell isolation and RNA extraction in woody plants' tissues, comprising the following steps:
- Collection of a woody plant tissue sample;
- Snap-freezing the sample in liquid nitrogen and embedding the sample in Optimal Cutting Temperature (OCT) freezing medium;
- Sectioning of the frozen samples into sections of 3 to 30 µm of thickness, at -10 to -27 °C;
- Fixation and dehydration of plant sections;
- Isolation of single cells by laser microdissection during a period of up to 180 min;
- RNA Extraction from isolated single-cells, wherein the RNA extraction comprises the use of a lysis buffer comprising stabilizers.

2. The method according to claim 1, wherein the OCT freezing medium comprises 10 to 12% Polyvinyl alcohol and 3,5 to 5% Polyethylene glycol, preferably 10,24% Polyvinyl alcohol and 4,26% Polyethylene glycol.

3. The method according to any of claims 1-2, wherein the laser microdissection is performed on PET-membrane slides

4. The method according to any of claims 1-3, wherein the frozen samples are sectioned at -25 °C.

5. The method according to any of claims 1-4, wherein frozen samples are sectioned into sections of 14 µm of thickness.

6. The method according to any of claims 1-5 the period of laser microdissection session for collecting cells should not be more than 40 minutes.

7. The method according to any of claims 1-6, wherein isolation of single cells by laser microdissection is performed until 18000 to 125000 cells are isolated.

8. The method according to any of claims 1-7, wherein the stabilizer in the lysis buffer used in the RNA extraction step comprises 1,5 to 5% of polyvinylpyrrolidone (PVP).
